# EUROPÄISCHE PATENTSCHRIFT

(11) **EP 1 277 055 B1**
(45) Veröffentlichungstag und Bekanntmachung des Hinweises auf die Patenterteilung: **30.04.2008**
(21) Anmeldenummer: 01913879.1
(22) Anmeldetag: 14.03.2001
(51) Int. Cl.: G01N 33/543

(54) **BIOCHIP ZUR ARCHIVIERUNG UND LABORMEDIZINISCHEN ANALYSE VON BIOLOGISCHEM PROBENMATERIAL**
BIOCHIP FOR THE ARCHIVING AND MEDICAL LABORATORY ANALYSIS OF BIOLOGICAL SAMPLE MATERIAL
BIOPUCE POUR L'ARCHIVAGE ET L'ANALYSE EN LABORATOIRE MEDICAL D'UN ECHANTILLON BIOLOGIQUE

(30) Priorität: 27.04.2000 DE 10020704
(43) Veröffentlichungstag der Anmeldung: 22.01.2003
(73) Patentinhaber: Bioref GmbH, 63776 Mömbris (DE)
(72) Erfinder: STAAB, Hans-Jürgen, 82319 Starnberg (DE)
(74) Vertreter: Flaccus, Rolf-Dieter
(86) Internationale Anmeldenummer: PCT/EP2001/002851
(87) Internationale Veröffentlichungsnummer: WO 2001/084150

(56) Entgegenhaltungen:
- WO-A1-97/45730
- DE-U- 29 616 373
- US-A- 4 959 308
- US-A- 5 972 626
- MENDOZA L G ET AL: "High-throughput microarray-based enzyme-linked immunosorbent assay (ELISA)." BIOTECHNIQUES, Bd. 27, Nr. 4, Oktober 1999 (1999-10), Seiten 778-788, XP000992893 ISSN: 0736-6205 in der Anmeldung erwähnt
- KONONEN J. ET AL: 'TISSUE MICROARRAYS FOR HIGH-THROUGHPUT MOLECULAR PROFILING OF TUMOR SPECIMENS' NATURE MEDICINE Bd. 4, Nr. 7, Juli 1998, NATURE PUBLISHING GROUP, NEW YORK, NY, US, Seiten 844 - 847, XP002927997

## Beschreibung

Die Erfindung betrifft einen für diagnostische Zwecke geeigneten Biochip, welcher mit dem zu analysierenden biologischen Probenmaterial beschichtet ist und sich sowohl für die raumsparende Archivierung von Probenmaterial als auch für dessen labormedizinische diagnostische Analyse eignet. Die Erfindung betrifft ferner Probenträger, welche für die Herstellung solcher Biochips geeignet sind, sowie Verfahren zur Herstellung der genannten Probenträger oder Biochips. Des weiteren betrifft die Erfindung diagnostische Verfahren unter Verwendung der erfindungsgemäßen Biochips, sowie die Verwendung der erfindungsgemäßen Biochips oder der genannten diagnostischen Verfahren in verschiedenen medizinischen Bereichen.

Die labormedizinische Diagnostik bildet eine wichtige Grundlage für medizinische Behandlungen. Durch die zunehmende Anzahl der zur Verfügung stehenden diagnostischen Marker-Moleküle werden die Möglichkeiten der labormedizinischen Diagnostik ständig erweitert. Wegen des Umfanges der vorzunehmenden Nachweisreaktionen, der hohen Dringlichkeit sowie aus wirtschaftlichen Gründen werden bevorzugt automatisierte Analyseverfahren eingesetzt, welche eine große Anzahl von verschiedenen Analysen in kurzer Zeit bewältigen können.

Ausgangsmaterial für die labormedizinische Analyse des Gesundheitszustandes eines Patienten sind in der Regel Körperflüssigkeiten, wie Vollblut, Plasma, Serum, Urin, Ascites, Fruchtwasser, Speichel, Liquor usw., oder Gewebeproben der verschiedenen Organe. Der behandelnde Arzt entnimmt dem Patienten die Probe, unterwirft diese ggf. einer speziellen Behandlung, z. B. durch Zentrifugation, und überführt die Patientenprobe danach in ein Probenröhrchen, in welchem die Probe versendet oder bis zur Durchführung der Analyse gelagert werden kann.

Abhängig vom zu analysierenden Parameter, d. h. der zu analysierenden Komponenten (Analyten), kann die Probe entweder bei Raumtemperatur, oder sie muß gekühlt oder in gefrorenem Zustand gelagert werden. Bei Langzeitlagerung, d. h. bei Lagerung über eine Zeitdauer von mehreren Wochen, Monaten oder Jahren, müssen die Patientenproben bei -20°C oder bei tieferer Temperatur eingefroren werden, um eine Degradation der Analyte zu vermeiden. Hauptursache für die bei Raumtemperatur auftretende Degradation sind die in dem flüssigen Probenmaterial enthaltenen Enzymaktivitäten.

Typische Probenvolumina für die Langzeitlagerung liegen bei mindestens 500 µl. Falls nach einer ersten Analyse zu einem späteren Zeitpunkt oder zu unterschiedlichen späteren Zeitpunkten weitere analytische Bestimmungen durchgeführt werden sollen, dann müssen, ausgehend von der ursprünglich erfolgten Blutabnahme, mehrere 500-µl-Proben hergestellt und aufbewahrt bzw. tiefgefroren gelagert werden. Diese Proben nehmen relativ viel Platz in Anspruch, wodurch eine Lagerung über einen längeren Zeitraum relativ teuer wird. Aus diesem Grund wird eine Langzeitlagerung von Patientenproben in der Regel nicht praktiziert. Dadurch muß allerdings auf die Möglichkeit verzichtet werden, zu einem späteren Zeitpunkt auf eine früher entnommene Probe zurückgreifen zu können. Dies ist in vielen Fällen wünschenswert oder sogar notwendig, wenn es darauf ankommt, den aktuellen Zustand eines Patienten, bezogen auf einen bestimmten diagnostischen Parameter, mit einem früheren Zustand bei demselben Patienten zu vergleichen. Gegebenenfalls kann es auch sinnvoll sein, mehrere frühere Zustände eines Patienten von verschiedenen Zeitpunkten zu erfassen, um eine Trendanalyse für den betreffenden Parameter durchzuführen. Solche Vergleiche sind allerdings nicht möglich, wenn der/die betreffende(n) diagnostische(n) Test(s) zu dem/den fraglichen früheren Zeitpunkt(en) nicht durchgeführt wurde(n) oder werden konnte(n), und eine Aufbewahrung der ursprünglichen Probe(n) nicht erfolgt war.

Für die Analyse des Gesundheitszustandes eines Patienten werden in der Labormedizin derzeit verschiedenste Verfahren angewandt. Hierzu gehören vor allem die Aktivitätsbestimmung von Enzymen, spezielle Färbereaktionen, immunchemische Verfahren, cytologische verfahren und molekularbiologische Verfahren. In den letzten Jahren haben vor allem die immunchemischen Verfahren an Bedeutung gewonnen und viele konventionelle Verfahren abgelöst. Auch molekularbiologische Verfahren finden verstärkt Eingang in die Routinediagnostik.

Die gegenwärtig verwendeten immunchemischen Analysensysteme basieren auf Antigen-Antikörper-Reaktionen, welche meist in einem Volumen von ca. 10-500 µl ablaufen. Dabei wird die Patientenprobe (Körperflüssigkeit etc.), welche den zu bestimmenden Analyten, in diesem Fall ein Antigen, enthält, zusammen mit einem für diesen Parameter spezifischen Antikörper, welcher nur diesen Analyten erkennt und bindet, inkubiert. Das Produkt aus dieser Antigen-Antikörper-Reaktion ist ein Komplex, welcher Antikörper-gebundene Antigene enthält. Je höher die Antigen-Konzentration in einer Patientenprobe ist, desto höher ist auch die Konzentration an gebildeten Antigen-Antikörper-Komplexen. Diese Antigen-Antikörper-Reaktionen finden in derzeitigen Testsystemen entweder frei in Lösung statt (Nachweis mittels Turbidimetrie oder Nephelometrie), oder sie werden auf antigenspezifischen Oberflächen durchgeführt (z. B. RIA, ELISA). Nach erfolgter Antigen-Antikörper-Reaktion befinden sich im ersten Fall die Antigen-Antikörper-Komplexe in Lösung, im zweiten Fall sind sie an eine Festphase, meist eine Kunststoffoberfläche, gebunden. Die Detektion und Quantifizierung von Antigen-Antikörper-Komplexen erfolgt bei der Turbidimetrie bzw. Nephelometrie durch Trübungsmessung, beim RIA (Radioimmunoassay) durch Radioisotop-markierte Antikörper in Verbindung mit radiometrischer Detektion, beim ELISA (enzyme-linked immunosorbent assay) und LIA mit enzymmarkierten Antikörpern in Verbindung mit der Detektion enzymkatalysierter Farbreaktionen. Mittels dieser Testsysteme lassen sich Analyt- bzw. Antigenkonzentrationen im Bereich von bis zu 1 pg/ml nachweisen (Protein).

An der Miniaturisierung und Automatisierung der oben genannten, in der Labortechnik etablierten Analysesysteme wird derzeit intensiv geforscht. So sind im Laufe der letzten Jahre verschiedene miniaturisierte, festphasen-gebundene Testsysteme entwickelt worden, welche wegen ihrer geringen Größe in Anlehnung an Computer-Chips als "Biochips" bezeichnet werden. Die Größe eines solchen Biochips liegt typischerweise zwischen 0,25 und 9 cm². In der Literatur beschriebene Biochips bestehen aus einer festen Matrix aus Glas (S.P.A. Fodor et al.: Light-directed, spatially addressable parallel chemical synthesis; Science 251 (Feb. 1991), S. 767-773); C.A. Rowe et al.: An array immunosensor for simultaneous detection fo clinical analytes; Analytical Chemistry Vol. 71 (Jan. 15, 1999) S. 433-439; L.G. Mendoza et al.: High-throughput microarray-based enzyme-linked immunosorbent assay (ELISA); BioTechniques 27 (Oct. 1999), S. 778-788), Nylon- oder Nitrocellulose-Membran, Silikon (J. Cheng et al.: Chip PCR. Nucleic Acids Research Vol. 24 (1996) S. 380-385) oder Silicium. Über verschiedene Linker-Moleküle können Biomoleküle, z. B. DNA, Peptide oder Proteine, kovalent an diese Matrices gekoppelt werden. Auf einer Fläche von 3,6 cm² können bis zu 10000 verschiedene Biomoleküle aufgebracht werden, was eine mikrometergenaue Adressierung der Biomoleküle auf spezielle, voneinander getrennte Areale der Matrix erfordert. Dies kann beispielsweise durch eine photolithographisch gesteuerte Synthese der Biomoleküle (S.P.A. Fodor et al.: Light-directed, spatially addressable parallel chemical synthesis; Science 251 (Feb. 1991), S. 767-773) oder durch Aufspotten der Biomoleküle mittels feinmechanischer, mikroprozessorgesteuerter programmierbarer Pipettierroboter erreicht werden (L.G. Mendoza et al.: High-throughput microarray-based enzyme-linked immunosorbent assay (ELISA); BioTechniques 27 (Oct. 1999), S. 778-788; M. Eggers et al.: A microchip for quantitative detection of molecules utilizing luminiscent and radioisotope reporter groups; BioTechniques 17 (1994) S. 516-523).

Mittels der festphasengebundenen Biomoleküle können anschließend die in Patientenproben enthaltenen, zu analysierenden Analyte gebunden und mit geeigneten Detektionssystemen nachgewiesen und quantifiziert werden.

Als Detektionssysteme mit hoher Empfindlichkeit werden z. B. charge-coupled-device(CCD)-Kameras (L. G. Mendoza et al., M. Eggers et al.; a. a. O.), Phototransistoren (T. Vo-Dinh et al.: DNA biochip using a phototransistor integrated circuit; Analytical Chemistry 71 (1999) S. 358-363) und Fluoreszenz-Detektoren (S.P.A. Fodor et al.; a. a. O.) verwendet.

Die oben beschriebenen Biochips werden zur Zeit ausschließlich für Forschungszwecke eingesetzt, z. B. für die DNA-Sequenzierung, die Gen-Expressionsanalyse, die Genmutationsanalyse und Proteinbindungsstudien, z. B. Antikörperbindungsstudien.

Bei der Genexpressionsanalyse (M. Schena et al.: Quantitative monitoring of gene expression patterns with a complementary DNA microarray; Science 270 (20 Oct 1995) S. 467-470) werden DNA-Sequenzen, die zu bestimmten Genen komplementär sind, mit Hilfe eines Pipettierroboters auf die Matrix eines Chip-Rohlings aufgespottet, wobei jeder einzelne Spot ein bestimmtes Gen repräsentiert. Anschließend wird aus der zu untersuchenden Gewebeprobe mRNA isoliert, mit einem Fluoreszenzfarbstoff markiert und auf den gesamten Biochip aufgetragen. Danach kann die Bindung von markierten mRNA-Molekülen an bestimmten Stellen des Biochips nachgewiesen werden. Nach erfolgter Analyse wird der Biochip verworfen, da er mit der mRNA-Probe kontaminiert ist.

Auf ähnliche Weine können Biochips zur DMA-Sequenzanalyse und zur Genmutationsanalyse verwendet werden.

Für die immunchemische Analyse von Probenmaterial ist von Mendoza et al. (a. a. O.) ein Prototyp eines miniaturisiertes Testsystems beschrieben worden. Die Autoren verwendeten eine spezielle Glasobjektträger mit 96 Vertiefungen ("wells"), wobei jede Vertiefung mittels eines Pipettierroboters und eines Kapillar-Druckverfahrens mit einem Punktmuster, bestehend aus 144 verschiedenen Antigenen, bedruckt wurde. Nach Inkubation mit antikörperhaltiger Lösung können diejenigen Punkte, an denen Antigen-Antikörper-Komplexe gebildet wurden, mittels einer CCD-Kamera nachgewiesen werden.

WO-A-97/45730 beschreibt Hochdurchsatz-Screening-Verfahren, bei welchen miniaturisierte Zell-Arrays verwendet werden. Bei diesen Screening-Verfahren wird die physiologische Reaktion von Zellen auf Testsubstanzen getestet. Die Zell-Arrays werden mit den Testsubstanzen kontaktiert, und anschließend eine computergestützte Auswertung der erhaltenen Signale vorgenommen. Als Zellen kommen beispielsweise Lymphozyten oder Krebszellen in Betracht.

Somit beschränken sich die aus dem Stand der Technik bekannten Biochips auf solche Ausführungsformen, bei denen spezifische, ausgewählte oder speziell synthetisierte Moleküle auf einer festen Matrix in einer definierten, miniaturisierten Anordnung aufgetragen und gebunden worden. Diese Moleküle dienen dazu, in einem heterogenen Gemisch, z. B. der Patienten-Probe, die Anwesenheit bestimmter Analyte nachzuweisen. Das bedeutet, daß diese Biochips testspezifisch sind, d. h. sie ermöglichen nur diejenigen Nachweisreaktionen, die durch die auf dem Chip befindlichen Nachweis-Moleküle vorgegeben sind.

Diese Vorgehensweise ist im Hinblick auf labordiagnostische Anwendung mit verschiedenen Nachteilen verbunden. Zwar ist es dadurch möglich, eine Vielzahl von verschiedenen Bestimmungen gleichzeitig vorzunehmen, allerdings ist für eine konkrete Fragestellung meist nur eine limitierte Anzahl von Parametern, d. h. von Analyten, relevant. Dies bedeutet, daß die Mehrzahl der auf einem solchen Chip befindlichen Nachweis-Moleküle ungenutzt bleibt oder nicht von Interesse ist. Zudem ist zu bedenken, daß ein derartiger Biochip nach Inkubation mit der Patientenprobe in der Regel unbrauchbar für weitere Analysen ist. Aus diesen Gründen ist die Verwendung von Biochips der beschriebenen Art für Routinenachweisverfahren in der Labormedizin wenig geeignet und nicht kosteneffektiv einzusetzen.

Ferner wird bei den bisher bekannten Biochips für jede weitere durchzuführende Analyse erneut Patienten-Probenmaterial benötigt. Dies hat zur Folge, daß der Patient gegebenenfalls mehrmals zur Blutentnahme erscheinen muß, was sowohl für den Arzt als auch für den Patienten mit gewissem Aufwand verbunden ist. Falls eine bestimmte Untersuchung mit einem bereits gewonnenen Probenmaterial zu einem späteren Zeitpunkt wiederholt werden soll, ist eine zwischenzeitliche Lagerung des Probenmaterials in flüssigem bzw. gefrorenem Zustand erforderlich, mit den eingangs beschriebenen Nachteilen.

Infolge der fortschreitenden Entwicklung werden laufend neuartige Nachweisreaktionen zugänglich gemacht. Um die aus dem Stand der Technik bekannten Biochips für solche neuentwickelten Nachweisreaktionen einsetzbar zu machen, müßten diese ständig aktualisiert werden, was ziemlich aufwendig ist und zudem nur mit einer gewissen zeitlichen Verzögerung möglich ist.

Häufig steht zum Zeitpunkt des Verfügbarwerdens einer neuartigen Nachweismethode das ursprüngliche Probenmaterial eines bestimmten Patienten nicht mehr zur Verfügung, so daß eine zeitliche Veränderung des interessierenden labormedizinischen Parameters meist nicht untersucht werden kann, da die längerfristige Aufbewahrung von Patienten-Probenmaterial problematisch ist. Die im Stand der Technik bekannten Biochips können zur Lösung dieses Problems keinen Beitrag leisten.

Die der Erfindung zugrundeliegende Aufgabe bestand deshalb darin, ein miniaturisiertes Analysensystem in Form eines Biochips für diagnostische Zwecke zur Verfügung zu stellen, welches die mehrmalige Durchführung von analytischen Nachweisverfahren, auch in größeren zeitlichen Abständen, an demselben Patienten-Probenmaterial ermöglicht. Ferner soll dieser Biochip eine platzsparende Aufbewahrung und Archivierung des Probenmaterials über einen längeren Zeitraum hinweg ermöglichen.

Ferner bestand die der Erfindung zugrundeliegende Aufgabe darin, ein Verfahren bereitzustellen, welches die automatisierte Durchführung von Nachweisreaktionen mit einem Biochip, der die obigen Voraussetzungen erfüllt, ermöglicht. Das Verfahren sollte in hohem Maße flexibel sein, d. h. leicht auf andere, ursprünglich nicht vorgesehene Nachweisreaktionen adaptierbar sein. Außerdem sollte das Verfahren die wiederholte Untersuchung desselben Probenmaterials in zeitlichen Abständen ermöglichen.

Überraschenderweise hat sich herausgestellt, daß die genannten Probleme durch ein diagnostisches Nachweisverfahren nach Anspruch 1, durch die Verwendung eines Biochips oder Probenträgers nach Anspruch 16 bzw. 17, und durch einen Biochip nach Anspruch 19 gelöst werden. Die Unteransprüche betreffen weitere Ausführungsformen der Erfindung, welche ebenfalls die genannte Aufgabe lösen.

Gemäß Anspruch 1 bezieht sich die vorliegende Erfindung auf ein diagnostisches Nachweisverfahren, welches die folgenden Schritte umfaßt:
a) Gewinnung von biologischem Probenmaterial in Form von Körperflüssigkeiten oder Gewebeproben aus einem zu untersuchenden Organismus, wobei es sich hierbei um einen einzigen, bestimmten individuellen biologischen Organismus handelt, vorzugsweise um einen individuellen Patienten;
b) Herstellung eines Biochips durch Bindung des Probenmaterials an die Oberfläche eines Probenträgers durch Beschichten mit dem flüssigen oder suspendierten Probenmaterial, erhalten aus Körperflüssigkeiten oder Zell- oder Gewebeproben, und nachfolgender Trocknung, wobei der Probenträger ein Probenträger aus einer festen Matrix ist und die Oberfläche des Probenträgers in Mikroareale untergliedert ist;
c) Auftragen von spezifischen, diagnostischen Nachweisreagenzien auf einzelne Mikroareale des Biochips unter Verwendung eines Pipettierroboters, und Inkubation unter den für die jeweilige Nachweisreaktion geeigneten Bedingungen;
d) Auftragen von Waschlösungen zur Unterdrückung unspezifischer Reaktionen, und nachfolgendem Absaugen dieser Waschlösungen;
e) falls erforderlich, Wiederholung der Schritte c) und d);
f) Detektion der Meßsignale von positiv reagierenden Mikroarealen;
g) computergestützte Auswertung der Meßdaten sowie Speicherung der Meßdaten.

Die vorliegende Erfindung betrifft ferner die Verwendung eines Biochips, welcher einen Probenträger aus einer festen Matrix umfaßt, für diagnostische Zwecke sowie zur Aufbewahrung und Archivierung des Probenmaterials. Die Oberfläche des Probenträgers ist in Mikroareale untergliedert, und auf der Oberfläche des Biochips ist ein zu analysierendes Probenmaterial gebunden, das nur von einem einzigen, bestimmten individuellen biologischen Organismus, vorzugsweise von einem individuellen Patienten, stammt.

Des weiteren betrifft die vorliegende Erfindung die Verwendung eines Probenträgers aus einer festen Matrix zur Aufbewahrung und Archivierung von aus Patienten gewonnenem Probenmaterial. Der Probenträger weist eine zur Bindung von biologischem Probenmaterial geeignete, in Mikroareale untergliederte Oberfläche auf.

im Gegensatz zu den durch den Stand der Technik bekannten Biochips sind im vorliegenden Fall nicht spezifisch ausgewählte oder synthetisierte Moleküle an die Chip-Oberfläche gebunden, sondern das Probenmaterial selbst, z. B. eine Körperflüssigkeit, welches eine heterogene Mischung verschiedener Biomoleküle darstellt. Der erfindungsgemäße Biochip stellt folglich einen patienten- oder patientenprobenspezifischen Chip dar, während es sich bei den bisher bekannten Biochips um testspezifische, d. h. nachweisspezifische Biochips handelt.

Die Bindung des Probenmaterials kann direkt an die Oberfläche der Matrix des Probenträgers erfolgen. Vorzugsweise ist die Matrix bzw. die Oberfläche des Probenträgers durch chemische oder physikalische Verfahren vorbehandelt, um die Bindungsfähigkeit der Oberfläche für das Probenmaterial zu verbessern. Geeignete Methoden hierfür sind dem Fachmann bekannt, z. B. Ätzung oder Anrauhen.

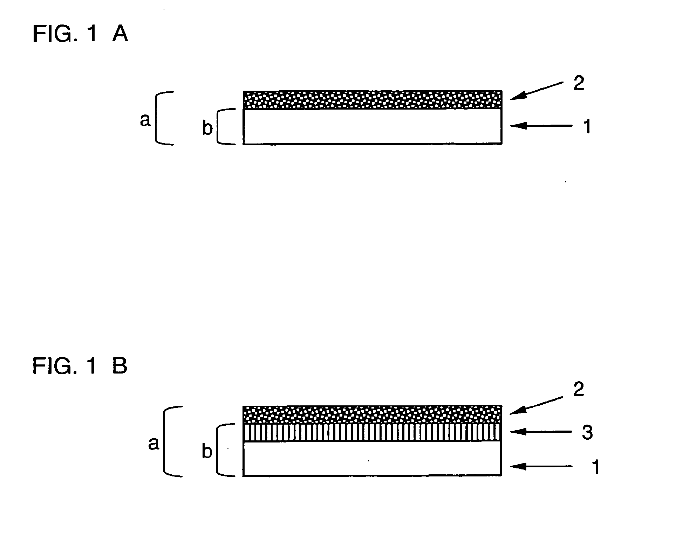

Der allgemeine Aufbau der erfindungsgemäßen Biochips ist aus Fig. 1 A ersichtlich, welche einen Biochip (a) im Schnitt darstellt, bestehend aus einem Probenträger (b) und darauf gebundenem Probenmaterial (2). Der Probenträger besteht im wesentlichen aus einer festen Matrix (1).

Die Bindung des Probenmaterials kann optional auch mittels einer auf der Oberfläche des Probenträgers aufgebrachten Schicht von Linker-Molekülen (Linker-Schicht) erfolgen.

Der schematische Aufbau eines derartigen Biochips (a) ist aus Fig. 1 B ersichtlich, welche einen aus einer festen Matrix (1) und einer darauf befindlichen Linker-Schicht (3) bestehenden Probenträger (b) im Schnitt darstellt, auf dessen Oberfläche das Probenmaterial (2) über die Linker-Schicht gebunden ist.

Die diagnostische Nachweisreaktion wird bei den erfindungsgemäßen Biochips prinzipiell auf die Weise durchgeführt, daß die zum Nachweis geeigneten Moleküle auf sehr kleine Areale des mit dem Probenmaterial beschichteten Biochips aufgebracht werden, wie weiter unten ausführlich beschrieben wird. Auf diese Weise ist es möglich, gleichzeitig oder nacheinander verschiedene Areale der Oberfläche des Biochips mit unterschiedlichen diagnostischen Nachweisreagenzien zu behandeln.

Da bei den erfindungsgemäßen Biochips das zu analysierende Probenmaterial, und nicht die zum Nachweis dienenden Moleküle, an die Oberfläche des die Matrix des Biochips bildenden Probenträgers gebunden sind, ist die Verwendung und das Einsatzgebiet eines solchen Chips nicht herstellungsbedingt auf bestimmte ausgewählte diagnostische Moleküle beschränkt. Vielmehr erfolgt die Auswahl der sinnvollen oder benötigten diagnostischen Nachweisreagenzien erst während der Anwendung des Nachweisverfahrens. Dadurch wird eine hohe Anwendungsflexibilität erreicht.

Bei den erfindungsgemäßen Biochips ist das zu analysierende Probenmaterial auf der Oberfläche des Probenträgers gebunden. Das Probenmaterial liegt dabei im getrockneten Zustand vor und ist deshalb selbst bei Raumtemperatur stabil lagerfähig. Da es sich bei den erfindungsgemäßen Biochips um miniaturisierte Systeme handelt, wird auf diese Weise eine raumsparende und kostengünstige Aufbewahrung und Archivierung von Patienten-Probenmaterial ermöglicht. Auf diese Weise kann eine große Zahl der erfindungsgemäßen patientenspezifischen Biochips auf kleinem Raum aufbewahrt werden und steht für die Durchführung von mehrfachen analytischen Nachweisreaktionen zur Verfügung.

Die Archivierbarkeit, in Verbindung mit der Möglichkeit, mehrere Nachweisreaktionen an verschiedenen Stellen der Chip-Oberfläche unabhängig voneinander vorzunehmen, gestattet es, die Anwesenheit eines bestimmten diagnostischen Markers bei einem bestimmten Patienten oder in einer bestimmten Probe eines Patienten in verschiedenen Zeitabständen zu untersuchen. Insbesondere ist es von Vorteil, daß solche diagnostischen Untersuchungen selbst dann noch zu einem späteren Zeitpunkt möglich sind, wenn diese nicht ursprünglich, d. h. zum Zeitpunkt der Probenentnahme, geplant waren. Dies trifft besonders auf solche Nachweisreaktionen zu, die erst zu einem späteren Zeitpunkt entwickelt worden sind.

Vorzugsweise enthält jedes einzelne Exemplar eines erfindungsgemäßen Biochips Probenmaterial, welches jeweils aus einem bestimmten, individuellen biologischen Organismus gewonnen wurde, z. B. Probenmaterial, welches aus einem bestimmten Patienten stammt. Als weitere biologische Organismen, aus welchen geeignetes Probenmaterial gewonnen werden kann, kommen neben dem Menschen auch Tiere, Pflanzen, Pilze und Mikroorganismen in Frage.

Als Probenmaterial, welches an die Oberfläche des Biochips gebunden ist, werden vorzugsweise Körperflüssigkeiten verwendet, wie z. B. Vollblut, Plasma, Serum, Urin, Ascites, Fruchtwasser, Speichel, Liquor, Lavagematerial aus Körperhohlräumen oder bronchoalveoläre Lavage. Ebenso können Gewebe- oder Organproben, oder Bestandteile, Zellen, Fraktionen, Zellaufschluß-Material, Konzentrate oder Extrakte aus den genannten Flüssigkeiten oder Gewebe- oder Organproben verwendet werden. In bestimmten Fällen kann es angebracht sein, die Probe so vorzubehandeln, daß die interessierenden Analyten, sofern in der Probe vorhanden, darin angereichert werden. Beispielsweise werden für eine Serumanalyse die festen Bestandteile der Blutprobe durch Zentrifugation abgetrennt; anschließend kann das so gewonnene Serum als Probenmaterial auf die Oberfläche des Probenträgers aufgetragen und gebunden werden.

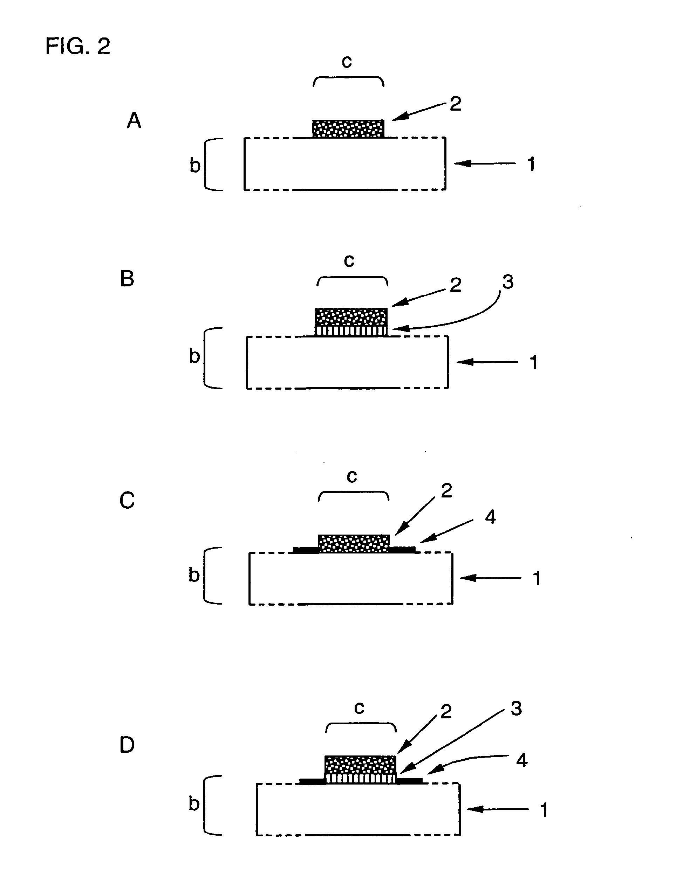

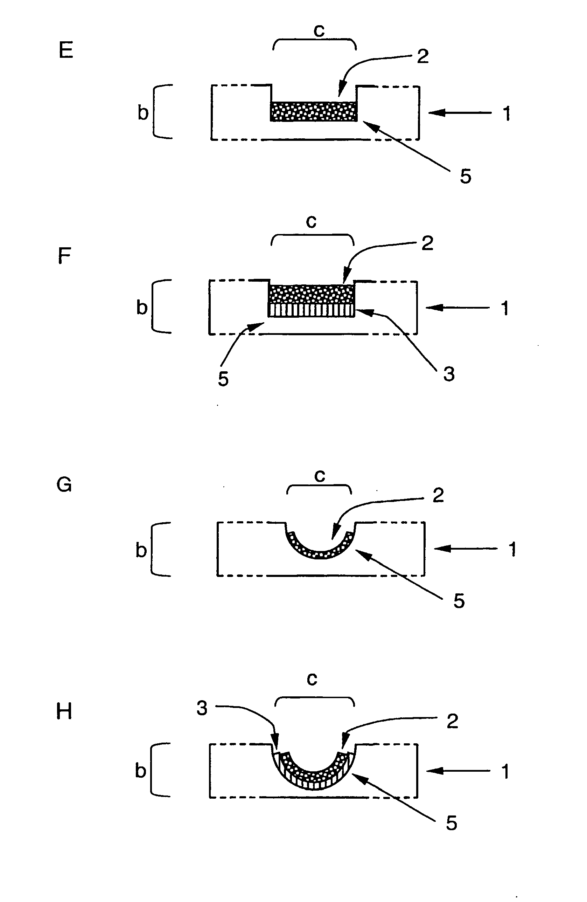

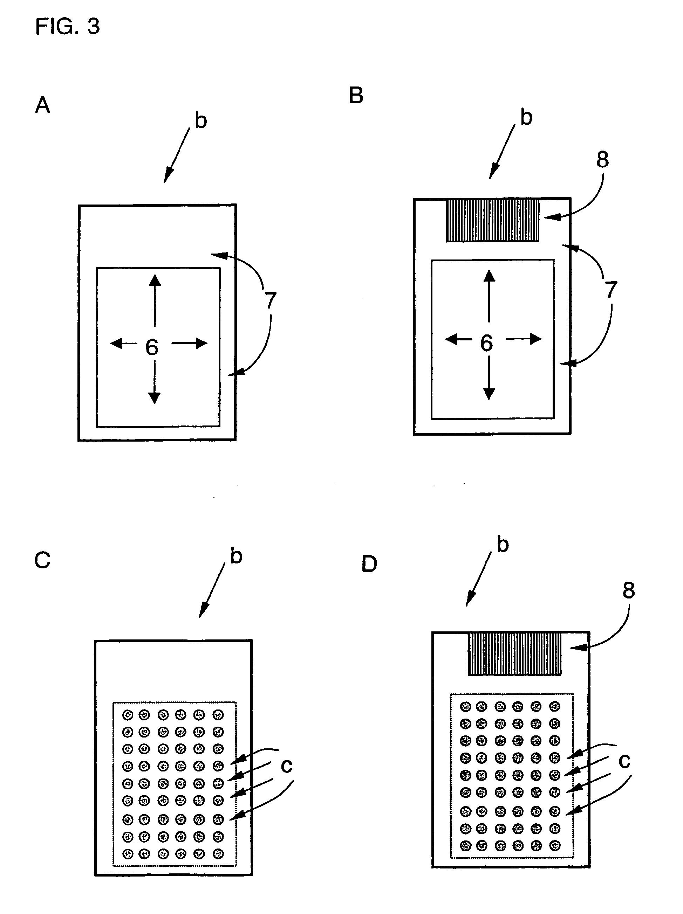

Bei einer besonders vorteilhaften Ausführungsform ist die Oberfläche des erfindungsgemäßen Probenträgers, welche mit dem Probenmaterial beladen ist, in Mikroareale untergliedert. Dabei handelt es sich um sehr kleine Flächenbereiche, die eng nebeneinander angeordnet sind, aber voneinander abgegrenzt sind. Üblicherweise ist die Oberfläche in eine Vielzahl solcher Mikroareale untergliedert, bevorzugt in mindestens 100 Mikroareale pro cm². Für bestimmte Anwendungszwecke kann auch eine geringere Anzahl von Mikroarealen pro Flächeneinheit ausreichend sein, z. B. 10 bis 100 Mikroareale pro cm². Die Mikroareale können vorzugsweise als Vertiefungen ausgestaltet oder durch hydrophobe oder nicht benetzbare Grenzbereiche voneinander abgegrenzt sein. Durch die Ausstattung des Probenträgers mit hydrophoben oder nicht benetzbaren Grenzbereichen kann erreicht werden, daß in diesen Bereichen keine Bindung von Probenmaterial stattfindet, so daß diese zwischen den einzelnen Mikroarealen befindlichen Grenzbereiche frei von Probenmaterial sind. Die Ausbildung der Mikroareale wird in den Fig. 2 und 3 weiter unten beispielhaft erläutert.

Durch die Unterteilung der Chip-Oberfläche in Mikroareale wird die Durchführung der analytischen Nachweisreaktionen verbessert, da durch diese Maßnahme das Auffinden und Behandeln einzelner Stellen durch einen Pipettierroboter erleichtert wird. Insbesondere, wenn die Mikroareale in Form eines regelmäßigen Gitters oder Rasters angeordnet sind, können einzelne Mikroareale anhand ihrer Koordinaten identifiziert und adressiert werden. Dadurch wird das selektive Auftragen von Nachweisreagenzien auf einzelne Stellen des Biochips unter Verwendung eines Pipettierroboters vereinfacht. Gleichzeitig wird durch die Unterteilung der mit Probenmaterial beschichteten Chip-Oberfläche die Nachweissicherheit erhöht bzw. das Risiko von unerwünschten Reaktionen oder Kontaminationen verhindert, da die einzelnen Mikroareale durch Grenzbereiche voneinander abgetrennt sind, oder in Form von Vertiefungen vorliegen.

Der Probenträger des erfindungsgemäßen Biochips, an dessen Oberfläche das Probenmaterial gebunden werden kann, besteht im wesentlichen aus einer festen Matrix. Als Grundmaterial für die Matrix des Probenträgers kommen beispielsweise Silikon, Kunststoffe (z. B. Polyvinylchlorid, Polyester, Polyurethane, Polystyrol, Polypropylen, Polyethylen, Polyethylentherephthalat, Polyamide, Nitrocellulose) oder Glas in Frage, jedoch können auch andere feste Materialien verwendet werden. Bevorzugt werden transparente feste Materialien verwendet. Allerdings ist bei der Auswahl des MatrixMaterials darauf zu achten, daß dieses für die dauerhafte Bindung des beabsichtigten Probenmaterials geeignet ist. Der Probenträger kann auch ein Laminat aus mindestens zwei unterschiedlichen Matrixmaterialien sein, wobei vorzugsweise eine der beiden Schichten eine starre Trägerschicht bildet, und die andere Schicht als Oberfläche für die Bindung des Probenmaterials dient.

Wie bereits erörtert, ist es besonders vorteilhaft, wenn die Oberfläche des Probenträgers, an welche das Probenmaterial gebunden werden soll, in Mikroareale untergliedert ist.

Die äußeren Abmessungen des erfindungsgemäßen Biochips werden so gewählt, daß das Erfordernis der Miniaturisierung erfüllt wird. Die Fläche eines solchen Biochips, oder eines Probenträgers für die Herstellung eines solchen Biochips, sollte höchstens 10 cm², vorzugsweise höchstens 4 cm², besonders bevorzugt höchstens 1 cm² betragen.

Bevorzugt ist der Probenträger bzw. der Biochip als flächiges Gebilde, besonders bevorzugt mit quadratischem oder rechteckigem Umriß, ausgestaltet. Aber auch andere geometrische Formen können in Frage kommen, z. B. runde bzw. kreisförmige Probenträger bzw. Biochips.

Die Dicke des flächigen Probenträgers beträgt vorzugsweise weniger als 3 mm, besonders bevorzugt weniger als 1 mm. Im Einzelfall können die äußeren Abmessungen des Biochips so gewählt werden, daß diese kompatibel zu bereits vorhandenen Pipettierrobotern bzw. Analysenautomaten sind und die Verwendung der erfindungsgemäßen Biochips in solchen Geräten ermöglichen.

Zur Herstellung eines erfindungsgemäßen Biochips wird die Oberfläche eines Probenträgers mit dem biologischen Probenmaterial beschichtet und dieses Material an die Oberfläche des Probenträgers gebunden, wobei es sich um eine kovalente oder nicht-kovalente Bindung handeln kann. Um bei Verwendung bestimmter Matrixmaterialien als Probenträger die Fähigkeit zur Bindung von Probenmaterial bzw. von Biomolekülen zu verbessern, kann es angebracht sein, diese Materialien bzw. die Oberfläche des Probenträgers einer Vorbehandlung mittels chemischer oder physikalischer Methoden zu unterziehen (z. B. Ätzung), wodurch das Bindungsvermögen erhöht wird. Des weiteren kann durch chemische oder physikalische Vorbehandlung die Oberfläche des Probenträgers vergrößert werden, welches zu einer Erhöhung der Bindungskapazität führt. Hierfür geeignete Methoden sind dem Fachmann bekannt.

Gemäß einer bevorzugten Ausführungsform der Erfindung kann die Oberfläche des Probenträgers vor dem Auftragen des Probenmaterials mit einer Linker-Schicht versehen werden, um die Bindung des Probenmaterials bzw. der darin enthaltenen Analyten (z. B. Biomoleküle oder Zellen) an die Oberfläche des Probenträgers zu verbessern, oder um eine selektive oder bevorzugte Bindung bestimmter Analyte oder Gruppen bzw. Klassen von Analyten zu bewirken. Auf diese Weise kann eine Anreicherung oder Vorselektion bestimmter Analyte oder Gruppen von Analyten erreicht werden. Als Linker werden dabei solche chemischen Verbindungen verstanden, die einerseits in der Lage sind, eine feste Bindung mit der Oberfläche des Probenträgers, d. h. mit dessen Matrixmaterial, einzugehen, und andererseits in der Lage sind, biologisches Probenmaterial zu binden oder selektiv bzw. bevorzugt zu binden.

Vorzugsweise kann die Oberfläche des Probenträgers mit einer Schicht aus Linker-Molekülen versehen werden, welche die selektive Bindung oder Anreicherung bestimmter Gruppen von biologischen Makromolekülen, vorzugsweise von Proteinen, Peptiden, Glykoproteinen, Zuckern, Lipiden, Nucleinsäuren, oder die Bindung oder Anreicherung von Zellen oder bestimmten Zelltypen oder Zellpopulationen ermöglichen. Ferner kann es auch vorteilhaft sein, wenn verschiedene Teilbereiche der Oberfläche eines Probenträgers mit unterschiedlichen Typen von Linkermolekülen beschichtet werden. Auf diese Weise ist es möglich, eine Vorselektion und räumliche Trennung der z. B. in einer Patientenprobe vorhandenen Analyten (z. B. verschiedene Klassen biologischer Makromoleküle) vorzunehmen.

Als Linker-Moleküle lassen sich beispielsweise (L.C. Shriver-Lake; Antibody immobilization using heterobifunctional crosslinkers; Biosensors & Bioelectronics Vol. 12 (1997), S. 1101-1106) einsetzen: N-succinimidyl-4-maleimidobutyrat (GMBS; zur Bindung von Aminogruppen), 4-(N-maleimidomethyl)-cyclohexan-1-carboxylhydrazid-HCl (M2C2H; zur Bindung von Zuckerresten), oder Antikörper mit bekannten Bindungsspezifitäten zur Bindung verschiedenster Makromoleküle oder Zellen bzw. Zelltypen.

Wie bereits erwähnt, ist es vorteilhaft, wenn die Oberfläche des Probenträgers bzw. des Biochips in Mikroareale unterteilt ist. Diese Unterteilung kann, außer durch die bereits beschriebenen Methoden, auch durch eine diskontinuierlich ausgebildete Linker-Schicht erreicht werden. Dabei werden Mikroareale aus linkerhaltigen Flächen und dazwischenliegenden linkerfreien Zonen gebildet, wobei vorzugsweise mindestens 100 Mikroareale pro cm² vorhanden sind.

Die Herstellung eines Probenträgers, dessen Oberfläche zur Bindung von Probenmaterial geeignet und vorzugsweise in Mikroareale unterteilt ist, kann auf verschiedene Weise erfolgen. Für die Herstellung von Flächenstücken mit den für Biochips geeigneten Ausmaßen wird das üblicherweise großformatig vorliegende, flächenförmige Matrix-Rohmaterial (Glas, Kunststoff etc.) mittels bekannter mechanischer Verfahren auf entsprechende Weise zerteilt, wodurch Probenträger erhalten werden. Alternativ kann das entsprechende Matrix-Rohmaterial auch verflüssigt werden und anschließend in entsprechende Formen gegossen werden, z. B. durch Spritzgießverfahren, um Probenträger herzustellen.

Die genannten Mikroareale bzw. die dazwischen liegenden Grenzbereiche lassen sich beispielsweise durch physikalische Methoden erzeugen, z. B. durch Gravur, Stanzung, Prägung oder Druck, oder durch chemische Methoden, wie Ätzung oder Auftragung hydrophober Schichten. Ferner ist es möglich, unter Zuhilfenahme eines Pipettierroboters Linker-Schichten in Form von Mikroarealen auf die Oberfläche von Probenträgern aufzutragen. Auch verschiedene bekannte Druckverfahren können adaptiert werden, um die Erzeugung der erfindungsgemäßen Mikroareale zu ermöglichen. Schließlich lassen sich die genannten Methoden auch kombiniert einsetzen. Ferner kann die Untergliederung in Mikroareale schon an dem flächig vorliegenden Matrix-Rohmaterial vorgenommen werden, welches anschließend auf beschriebene Weise in einzelne Probenträger zerteilt wird.

Die erfindungsgemäßen Biochips sind besonders für die Archivierung und mehrmalige diagnostische Analyse von Probenmaterial aus jeweils einem bestimmten Patienten geeignet ("patientenspezifischer" Biochip). Um die Archivierung solcher Chips und deren automatische Prozessierung und Auswertung in Analysenautomaten zu erleichtern bzw. zu ermöglichen, wird gemäß einer weiteren Ausführungsform der Erfindung vorgeschlagen, daß der Biochip bzw. ein entsprechender Probenträger mit Mitteln ausgestattet ist, die eine Identifizierung des Biochips oder die Speicherung von patientenbezogenen Daten oder die Speicherung von Analysendaten ermöglichen. Dies kann vorzugsweise mittels eines maschinenlesbaren Bar-Codes, eines maschinenlesbaren Magnetstreifens, eines digitalen Speicherelements oder eines anderen computerlesbaren Speichermediums erfolgen. Insbesondere wenn große Zahlen von patientenspezifischen Biochips archiviert oder prozessiert werden müssen, ist die vorstehend beschriebene Ausführungsvariante vorteilhaft.

Das Vorhandensein eines Mittels zur Datenspeicherung auf dem Biochip ermöglicht es beispielsweise dem Arzt oder Laborarzt, der die Probenentnahme und gegebenenfalls das Auftragen des Probenmaterials auf den Probenträger vornimmt, z. B. patientenbezogene Daten oder Daten zur Probenentnahme auf dem Biochip zu speichern. Ferner ist es dadurch auch möglich, Analysenergebnisse bzw. deren Auswertung zu speichern, so daß auch die Informationen über bereits früher durchgeführte Analysen gespeichert werden können.

Die Erfindung umfaßt ferner diagnostische Nachweisverfahren, welche Verfahrensschritte zur Herstellung eines Biochips unter Verwendung von Probenmaterial, sowie Verfahrensschritte zur Durchführung diagnostischer Nachweisreaktionen und zu deren Auswertung umfaßt.

Zunächst werden Körperflüssigkeiten, Gewebeproben oder dgl. aus einem Patienten oder einem anderen Organismus gewonnen und, falls erforderlich, einer Vorbehandlung unterzogen (z. B. Zentrifugation, Konzentration usw.). Falls erforderlich, wird das Probenmaterial in einem geeigneten Puffer oder Lösungsmittel suspendiert oder gelöst. Zur Herstellung eines Biochips wird dieses Probenmaterial, vorzugsweise in flüssiger Form, auf die Oberfläche eines erfindungsgemäßen Probenträgers aufgetragen, z. B. mittels Pipette oder durch Eintauchen. Nach einer kurzen Inkubationszeit von wenigen Minuten, z. B. 1 bis 10 min, wird gegebenenfalls das überschüssige Probenmaterial entfernt (z. B. durch Absaugen). Anschließend wird eine Trocknung durchgeführt, vorzugsweise bei leicht erhöhten Temperaturen (30-60 °C), wodurch die Bindung des Probenmaterials bzw. der darin enthaltenen Analyte (z. B. Biomoleküle) an die Oberfläche des Probenträgers oder gegebenenfalls an die Linker-Schicht erfolgt. Die vorstehend genannten Verfahrensschritte können von dem Arzt, welcher das Probenmaterial entnimmt, vorgenommen werden, oder von medizinischem Hilfspersonal.

Die so erhaltenen patienten- oder probenspezifischen Biochips können anschließend trocken bei Raumtemperatur gelagert werden und stehen für eine sofortige oder spätere Analyse bereit.

Zur Durchführung von analytischen Nachweisreaktionen an einem solchen mit Probenmaterial beschichteten Biochip werden in den nachfolgenden Schritten die gewünschten Nachweisreagenzien vorzugsweise unter Verwendung eines Pipettierroboters auf einzelne Stellen oder auf einzelne Mikroareale dieses Biochips aufgetragen. Als Nachweisreagenzien kommen beispielsweise Antikörper, Antigene, sequenzspezifische Antikörper, Lektine, DNA-Sonden, niedermolekulare Liganden, Hormone, Biomolekül-bindende Farbstoffe oder andere spezifisch bindende Moleküle in Frage.

Die genannten Stellen oder Mikroareale des Biochips werden für eine bestimmte Zeit und bei einer bestimmten Temperatur mit den gewählten Nachweisreagenzien in bestimmten Konzentrationen inkubiert, wobei die jeweils zu wählenden Versuchsparameter von der Art der verwendeten Nachweisreagenzien und dem jeweiligen Probenmaterial abhängen. Der Fachmann ist in der Regel mit der Auswahl geeigneter Versuchsparameter vertraut.

Nach Ende der festgelegten Inkubationszeit wird das flüssige Nachweisreagens mittels Pipettierroboter durch Absaugen wieder entfernt. Anschließend werden in einem nächsten Schritt, ebenfalls mittels Pipettierroboter, Waschlösungen und erforderlichenfalls weitere Nachweisreagenzien in sequentieller Folge auf die zu analysierenden Stellen bzw. Mikroareale des Biochips aufgetragen und danach wieder abgesaugt. Die dabei verwendbaren Waschlösungen und die zusätzlich einzusetzenden Nachweisreagenzien, z. B. fluoreszenzmarkierte Antikörper, sowie die geeigneten Reaktionsbedingungen, sind dem Fachmann grundsätzlich bekannt.

Schließlich werden diejenigen Stellen oder Mikroareale, in denen eine positive Nachweisreaktion stattgefunden hat, durch einen geeigneten Detektor identifiziert. Vorzugsweise erfolgt die Registrierung dieser Meßsignale mit Hilfe von CCD-Kameras, Phototransistoren oder Radioaktivitäts-, Luminiszenz- oder Fluoreszenzdetektoren, wobei sich die Auswahl der Detektionsmethode nach der Art der eingesetzten Nachweisreagenzien richtet. Die von den Detektoren gelieferten primären Meßdaten können anschließend einer computergestützten Auswertung unterworfen werden.

Die positiven Nachweisreaktionen kommen grundsätzlich durch eine spezifische Wechselwirkung oder Bindung zwischen den Analyten (z. B. bestimmte Antigene in der Patienten-Probe) und dem eingesetzten Nachweisreagens zustande, z. B. Bildung von Antigen-Antikörper-Komplexen. Diese werden durch weitere Reaktionsschritte bzw. Reagenzien, welche dem Fachmann bekannt sind, detektierbar gemacht.

Besonders vorteilhaft ist es, wenn eine größere Anzahl der erfindungsgemäßen Biochips, z. B. von verschiedenen Patienten, gleichzeitig oder parallel auf die beschriebene Weise analysiert werden, d. h. wenn die Nachweisreaktionen und die Detektion gleichzeitig oder parallel an mehreren Biochips durchgeführt wird.

Ferner ist es vorteilhaft, wenn der bzw. die Biochips parallel (gleichzeitig) oder sequentiell nacheinander mit verschiedenen Nachweisreagenzien mit unterschiedlichen Erkennungsspezifitäten behandelt werden, wobei diese Nachweisreagenzien jeweils an noch nicht vorher untersuchte Stellen oder Mikroareale des Biochips aufgetragen werden. Auf diese Weise läßt sich in kürzester Zeit die An- oder Abwesenheit bestimmter diagnostischer Markermoleküle in mehreren patientenspezifischen Biochips testen. Um zu verhindern, daß eine bestimmte Stelle oder Mikroareal eines Biochips mehrfach für Nachweisreaktionen herangezogen wird, kann vorgesehen sein, daß die Position der bereits "verbrauchten" Stellen oder Areale registriert und auf dem Speichermedium des Biochips gespeichert wird.

Des weiteren können auch unterschiedliche Stellen oder Mikroareale desselben Biochips mit dem gleichen Nachweisreagens, ggf. in unterschiedlichen Konzentrationen, analysiert werden. Auf diese Weise läßt sich durch Mehrfach- bzw. Parallelmessungen die Nachweissicherheit erhöhen.

Nach erfolgter Nachweisreaktion und Detektion können die Biochips wieder der Archivierung zugeführt und aufbewahrt werden. Sie stehen für weitere Nachweisreaktionen zur Verfügung. Dies bedeutet, daß derselbe Biochip, welcher mit Probenmaterial eines bestimmten Patienten beschichtet ist, zweimal oder mehrmals nacheinander einer Analyse, bestehend aus Nachweisreaktion, Detektion und Auswertung, nach den oben beschriebenen Schritten unterzogen werden kann, wobei der Biochip in der Zeit zwischen den aufeinanderfolgenden Analysen aufbewahrt bzw. archiviert wird. Dabei werden bei den aufeinanderfolgenden Analysen unterschiedliche Mikroareale des Biochips mit unterschiedlichen Nachweisreagenzien behandelt.

Durch die Möglichkeit, an jeweils einem Biochip, der mit Probenmaterial eines bestimmten Patienten beschichtet ist, eine Vielzahl von diagnostischen Nachweisreaktionen, auch in größerem zeitlichen Abstand, durchführen zu können, wird der Bedarf an Probenmaterial erheblich verringert, d. h. ausgehend von einer anfänglichen relativ kleinen Probenmenge (z. B. 0,5 ml) lassen sich Biochips herstellen, die eine große Anzahl von Nachweisreaktionen über einen längeren Zeitraum hinweg ermöglichen.

Die erfindungsgemäßen Biochips mit dem darauf gebundenen Probenmaterial sind im trockenen Zustand bei Raumtemperatur über mehrere Jahre, mindestens über einen Zeitraum von 5 Jahren hinweg lagerfähig und können während dieser Aufbewahrungszeit zwischenzeitlich mehrfach zur Durchführung der beschriebenen Nachweisreaktionen verwendet werden. Die erfindungsgemäßen Biochips sind deshalb immer dann von Vorteil, wenn es auf eine Aufbewahrung bzw. Archivierung von Probenmaterial aus Patienten, mit der Möglichkeit zur Durchführung diagnostischer Nachweisreaktionen ankommt.

In speziellen Fällen, wenn die Stabilität gewisser Analyte im Probenmaterial kritisch erscheint, kann auch eine Lagerung der Biochips bei tieferen Temperaturen, z. B. bei 0 °C bis 15 °C oder bei noch niedrigeren Temperaturen, z. B. unterhalb von 0 °C erfolgen. Grundsätzlich ist davon auszugehen, daß nach einer längerandauernden oder mehrjährigen Aufbewahrung der Biochips im trockenen Zustand eine weitgehende Inaktivierung von potentiell degradierenden Enzymen, wie Nukleasen oder Proteasen, stattgefunden hat.

Die Lagerung bzw. Archivierung der erfindungsgemäßen Biochips kann zweckmäßigerweise mit einem verschließbaren Kasten erfolgen, welcher an seinen inneren Seitenwänden Führungsschienen aufweist, entlang welcher die Biochips in den Kasten eingeführt werden können und mittels derer sie im Kasten arretiert sind. Durch Anbringen mehrerer solcher Führungsschienen lassen sich mehr als 100 Biochips platzsparend in einem einzigen Kasten aufbewahren. Eine größere Anzahl der genannten Kästen kann wiederum als fester oder beweglicher Bestandteil in ein Schubladensystem oder Schranksystem integriert werden.

Aufgrund ihrer Lagerfähigkeit und der Möglichkeit, eine große Zahl unabhängiger Nachweisreaktionen vorzunehmen, eignen sich die erfindungsgemäßen Biochips bzw. die erfindungsgemäßen diagnostischen Nachweisverfahren für eine Vielzahl von Nutzanwendungen. Im Folgenden werden einige beispielhafte Anwendungsmöglichkeiten beschrieben.

Die erfindungsgemäßen Biochips können im Bereich der Tumordiagnostik eingesetzt werden, um beispielsweise das Auftreten bestimmter Tumormarker im Serum oder in Gewebeproben zu beobachten. Diese Tumormarker werden von Tumoren gebildet und in Körperflüssigkeiten des Erkrankten sezerniert, oder sie werden vom Organismus infolge seiner Reaktion auf den Tumor gebildet. Hingegen fehlen diese Markermoleküle im Serum oder anderen Köperflüssigkeiten von Gesunden, oder sind dort nur in geringen Mengen vorhanden. Im Verlauf des Tumorwachstums kann es zu einem Ansteigen der Serumkonzentrationen des Markers kommen. Problematisch ist allerdings die Beurteilung "mittlerer" Serumkonzentrationen hinsichtlich einer Diagnose, da dieser Befund sowohl auf einen zwar abnormen, aber dennoch unbedenklichen Normalwert (z. B. genetisch bedingt) als auch auf ein beginnendes Tumorwachstum hindeuten kann. Entscheidend ist deshalb die Frage, ob zu einer bestimmten Zeit ein Anstieg der Serumkonzentration eines solchen Tumormarkers stattgefunden hat. Diese Frage läßt sich beantworten, wenn in bestimmten Zeitabständen die Serumkonzentration dieses Tumormarkers untersucht wird. Nur auf diese Weise läßt sich frühzeitig und mit relativer Sicherheit ein beginnendes Tumorwachstum erkennen. Grundsätzlich müssen bei einem jeden Probanden derartige Marker-Nachweise in zeitlichen Abständen durchgeführt werden (d. h. eine sequentielle Bestimmung), so daß eine Trendanalyse durchgeführt werden kann.

Die erwähnten Trendanalysen bzw. sequentiellen Bestimmungen von Tumormarkern unter Verwendung der erfindungsgemäßen Biochips können auch zur Verlaufskontrolle von Tumoren eingesetzt werden, z. B. zur postoperativen Rezidivkontrolle oder zur Kontrolle einer cytostatischen Therapie.

Mit den bekannten Archivierungsmethoden für Patientenproben lassen sich die genannten Testreihen nicht oder nur unter hohem Aufwand realisieren. Hingegen wird es durch die erfindungsgemäßen Biochips und dem erfindungsgemäßen Verfahren möglich, dieses Probenmaterial in großer Zahl und raumsparend längerfristig aufzubewahren, und eine Vielzahl von diagnostischen Nachweisreaktionen, z. B. zur Bestimmung von Tumormarkern, durchzuführen. Dadurch werden retrospektive Trendanalysen ermöglicht, welche die Diagnosefindung wesentlich beschleunigen. Durch die Archivierbarkeit der erfindungsgemäßen Biochips wird es auch möglich, bei neu bekannt werdenden Tumormarkern deren An- oder Abwesenheit in älteren Biochips eines bestimmten Probanden zu testen.

Die Erfindung kann somit dazu beitragen, das Wachstum von Tumoren frühzeitig zu erkennen und entsprechend zu intervenieren.

Des weiteren eignen sich die erfindungsgemäßen Biochips für Zwecke der klinischen Forschung, wo die Archivierung von Patienten-Probenmaterial ebenfalls eine wichtige Rolle spielt. Dies ist vor allem von Bedeutung, wenn - beispielsweise bei Änderung der wissenschaftlichen Fragestellung - Nacherhebungen an früheren Patientenproben erforderlich sind. Gegenüber der derzeitig verwendeten Methoden - Einfrieren von Proben (in der Regel mindestens 0,5 ml) und späteres Wiederauftauen - sind die erfindungsgemäßen Biochips viel besser geeignet, da sie eine wesentlich größere Zahl von Nachweisreaktionen bei gleichzeitig wesentlich geringerem Platzbedarf und einfacherer Lagerung ermöglichen. Wegen der großen Anzahl der Nachweisreaktionen, die ein Biochip ermöglicht, entfällt auch die Notwendigkeit, zu einem bestimmten Zeitpunkt mehrere parallele Proben eines Patienten herzustellen und zu lagern.

Ein weiterer Anwendungsbereich der erfindungsgemäßen Biochips sind Blutbanken oder Firmen oder andere Organisationen, die humanes Spendermaterial (z. B. Körperflüssigkeiten, Zellen, Gewebe) verarbeiten, und bei welchen Proben des Spendermaterials und gegebenenfalls von daraus hergestellten Produkten für diagnostische Nachweisreaktionen zu Kontrollzwecken aufbewahrt werden müssen oder sollten. Gewöhnlich werden lediglich die Analysenergebnisse, nicht aber die Proben selbst aufbewahrt, da dies zu viel Platz beanspruchen würde. Allerdings kann es im Zusammenhang mit auftretenden Kontaminationen auch von Vorteil sein, solche Proben einer erneuten Analyse, gegebenenfalls unter Verwendung anderer Nachweismethoden, zu unterziehen, z. B. zur rechtlichen Absicherung. Eine erneute Analyse solcher Proben kann auch vorteilhaft sein, wenn der Nachweis erbracht werden soll, daß ein Roh-, Zwischen- oder Endprodukt aus humanem Material in seiner Zusammensetzung den Vorgaben oder gesetzlichen Bestimmungen genügt hat. Die erfindungsgemäßen Biochips können vorteilhaft zur Archivierung und nachfolgenden Analyse von Proben aus Spenderblut oder anderem Spendermaterial von Blutbanken, Firmen und anderen Organisationen verwendet werden. Die archivierten Biochips ermöglichen es, im Falle einer aufgetretenen Kontamination, oder eines Verdachtes einer Non-Konformität, die verdächtigten Spenderproben oder Proben darauf basierender Produkte im Nachhinein zu analysieren. So kann im Nachhinein der Nachweis der Konformität einer Spender- oder Produktprobe mit den Vorgaben, Normen oder gesetzlichen Bestimmungen erbracht werden. Dies kann insbesondere bei juristischen Auseinandersetzungen von großer Bedeutung und Hilfe sein. Ferner können die erfindungsgemäßen Biochips oder Probenträger zur Aufbewahrung und Archivierung von Patienten-Probenmaterial für Zwecke der Routinediagnostik von Patientenproben verwendet werden.

Die erfindungsgemäßen Biochips lassen sich auch vorteilhaft bei epidemiologischen Studien einsetzen, beispielsweise um die Ausbreitung von Infektionskrankheiten zu untersuchen. Bisher scheiterten solche Untersuchungen oft an dem nicht mehr vorhandenen Probenmaterial aus der zurückliegenden Zeit, da es aus praktischen Gründen nicht möglich war, die erforderliche Anzahl von Blutproben oder dgl. aufzubewahren. Hingegen ermöglichen die hier beschriebenen Biochips eine raumsparende und kostengünstige Langzeitlagerung bzw. Archivierung. Damit kann eine große Anzahl von Proben archiviert werden. Diese Proben stehen für spätere epidemiologische Studien zur Verfügung; somit können diese Studien ein viel größeres Patientenkollektiv berücksichtigen, da sie auch auf archivierte Proben aus der Vergangenheit zurückgreifen können.

Die erfindungsgemäßen Biochips bzw. die erfindungsgemäßen Verfahren können im Zuammenhang mit verschiedenen Fragestellungen im Bereich der Medizin oder der Tiermedizin vorteilhaft eingesetzt werden. Neben den bereits genannten Anwendungsbeispielen kommen auch Anwendungen für Zwecke der Gewebstypisierung oder im Bereich der forensischen Medizin in Betracht.

Nachfolgend werden anhand der Fig. 1-3 sowohl das Grundprinzip sowie beispielhaft einige bevorzugte Ausführungsformen der Erfindung näher erläutert. Die Erfindung ist jedoch nicht auf die dargestellten Ausführungsformen beschränkt. Die Bezugszeichen werden in allen Figuren in gleicher Weise verwendet und haben jeweils die gleiche Bedeutung.
Fig. 1 A zeigt einen Schnitt durch einen erfindungsgemäßen Biochip (a), welcher aus einem Probenträger (b) und dem auf seiner Oberfläche gebundenen biologischen Probenmaterial (2) besteht. Der Probenträger (b) besteht im wesentlichen aus einer festen Matrix (1).
Fig. 1 B zeigt in Schnittdarstellung eine Ausführungsform des erfindungsgemäßen Biochips (a), bei welchem der Probenträger (b) eine feste Matrix (1) und zusätzlich eine darauf befindliche Linkerschicht (3) umfaßt. Das Probenmaterial (2) ist über die Linkerschicht (3) an die Matrix des Probenträgers (1) gebunden.
Fig. 2 zeigt Schnittdarstellungen von verschiedenen Ausführungsformen des erfindungsgemäßen Biochips, wobei diese Ausführungsformen die oben erwähnten Mikroareale (c) aufweisen. In den Fig. 2 A bis H ist jeweils ein Bereich eines Biochips dargestellt, in welchem sich ein solches Mikroareal befindet.
Fig. 2 A zeigt einen Schnitt durch einen Biochip im Bereich eines Mikroareals (c), wobei (b) wiederum den aus der festen Matrix (1) bestehenden Probenträger bezeichnet. Wie ersichtlich, ist das biologische Probenmaterial (2) nur im Bereich des Mikroareals (c) an die Oberfläche des Probenträgers gebunden. Die benachbarten Bereiche der Probenträger-Oberfläche sind frei von Probenmaterial.
Fig. 2 B zeigt ebenfalls einen Schnitt durch einen Biochip im Bereich eines Mikroareals (c), ähnlich wie Fig. 2 A, wobei jedoch das Probenmaterial (2) über eine Linkerschicht (3) an die Matrix (1) des Probenträgers (b) gebunden ist. Die Linkerschicht ist jeweils nur im Bereich eines Mikroareals vorhanden; die benachbarten Bereiche sind frei von Linkermolekülen.
Fig. 2 C zeigt ebenfalls einen Schnitt durch einen Biochip im Bereich eines Mikroareals (c), ähnlich wie Fig. 2 A, wobei allerdings die die Mikroareale jeweils umgebenden Grenzbereiche (4) mit hydrophoben Eigenschaften ausgestattet sind oder nicht benetzbar sind, so daß in diesen Grenzbereichen kein Probenmaterial (2) gebunden werden kann.
Fig. 2 D zeigt eine Ausführungsform in einer Darstellung wie in Fig. 2 A, bei welcher die in den Fig. 2 B und 2 C gezeigten Maßnahmen mit einander kombiniert werden. Das Probenmaterial (2) ist über eine im Bereich des Mikroareals (c) befindliche Linkerschicht (3) an die Matrix des Probenträgers gebunden. Zusätzlich sind die die Mikroareale jeweils umgebenden Grenzbereiche (4) mit hydrophoben Eigenschaften ausgestattet oder unbenetzbar gemacht worden.
Fig. 2 E zeigt eine weitere Ausführungsform in einer Darstellung wie in Fig. 2 A, wobei ein Mikroareal (c) gezeigt ist, welches in Form einer Vertiefung (5) ausgestaltet ist, in welcher das Probenmaterial (2) an die Matrix (1) des Probenträgers (b) gebunden ist.
Fig. 2 F zeigt eine Variation der in Fig. 2 E gezeigten Ausführungsform, wobei ein Mikroareal (c) gezeigt ist, welches in Form einer Vertiefung (5) ausgebildet ist, deren Boden mit einer Linkerschicht (3) beschichtet ist, mittels welcher das Probenmaterial (2) an die Matrix (1) des Probenträgers (b) gebunden werden kann.
Fig. 2 G zeigt eine Variante der in Fig. 2 E gezeigten Ausführungsform, wobei die Vertiefung (5) des Mikroareals (c) in Form eines gerundeten Napfes gestaltet ist.
Fig. 2 H zeigt eine Variante der in Fig. 2 F gezeigten Ausführungsform, wobei die Vertiefung (5) des Mikroareals (c) in Form eines gerundeten Napfes gestaltet ist.
Fig. 3 zeigt beispielhaft einige Ausführungsformen der erfindungsgemäßen Biochips bzw. Probenträger in der Aufsicht.
Fig. 3 A zeigt die Grundform eines erfindungsgemäßen Biochips bzw. Probenträgers (b), wobei die zur Beschichtung mit Probenmaterial vorgesehene Fläche mit (6) bezeichnet ist. Die außerhalb der Fläche (6) befindlichen Bereiche, insbesondere die Randbereiche, können hydrophob oder nicht benetzbar ausgestattet sein.
Fig. 3 B zeigt eine Variation des in Fig. 3 A gezeigten Probenträgers (b), welcher zusätzlich (8) mit einem Barcode, Magnetstreifen oder einem sonstigen Speichermedium versehen ist.
Fig. 3 C zeigt einen Probenträger (b) bzw. Biochip in der Aufsicht, wobei beispielhaft die Anordnung der erwähnten Mikroareale (c) dargestellt ist. Wie in Fig. 3 A und B können auch hier die Randbereiche hydrophob oder nicht benetzbar ausgestattet sein.
Fig. 3 D zeigt schließlich eine Variante der in Fig. 3 C vorgestellten Ausführungsform, bei welcher - wie in Fig. 3 B - zusätzlich ein Mittel zur Datenspeicherung vorgesehen ist, z. B. Barcode, Magnetstreifen oder ein anderes Speichermedium.

## Patentansprüche

1. Diagnostisches Nachweisverfahren, welches die folgenden Schritte umfaßt:
a) Bereitstellung von biologischem Probenmaterial in Form von Körperflüssigkeiten oder Gewebeproben aus einem zu untersuchenden Organismus, wobei es sich hierbei um einen einzigen, bestimmten individuellen biologischen Organismus handelt, vorzugsweise um einen individuellen Patienten;
b) Herstellung eines Biochips durch Bindung des Probenmaterials an die Oberfläche eines Probenträgers durch Beschichten mit dem flüssigen oder suspendierten Probenmaterial, erhalten aus Körperflüssigkeiten oder Zell- oder Gewebeproben, und nachfolgender Trocknung, wobei der Probenträger ein Probenträger aus einer festen Matrix ist und die Oberfläche des Probenträgers in Mikroareale untergliedert ist;
c) Auftragen von spezifischen, diagnostischen Nachweisreagenzien auf einzelne Mikroareale des Biochips unter Verwendung eines Pipettierroboters, und Inkubation unter den für die jeweilige Nachweisreaktion geeigneten Bedingungen;
d) Auftragen von Waschlösungen zur Unterdrückung unspezifischer Reaktionen, und nachfolgendem Absaugen dieser Waschlösungen;
e) falls erforderlich, Wiederholung der Schritte c) und d);
f) Detektion der Meßsignale von positiv reagierenden Mikroarealen;
g) computergestützte Auswertung der Meßdaten sowie Speicherung der Meßdaten.

2. Verfahren nach Anspruch 1, **dadurch gekennzeichnet, daß** in Schritt a) das biologische Probenmaterial vor der Bindung an die Oberfläche des Probenträgers aufbereitet wird, vorzugsweise durch Zentrifugation, Zellaufschluß oder Extraktion.

3. Verfahren nach Anspruch 1, **dadurch gekennzeichnet, daß** die Schritte c) bis g) an mehreren Biochips parallel oder gleichzeitig durchgeführt werden.

4. Verfahren nach Anspruch 1, **dadurch gekennzeichnet, daß** einzelne Mikroareale eines Biochips gleichzeitig oder sequentiell mit verschiedenen spezifischen diagnostischen Nachweisreagenzien behandelt werden.

5. Verfahren nach Anspruch 1, **dadurch gekennzeichnet, daß** als spezifische diagnostische Nachweisreagenzien in Schritt c) vorzugsweise Antikörper, Antigene, Lektine, DNA-Sonden, Biomolekül-bindende Farbstoffe oder andere spezifisch bindende Moleküle eingesetzt werden.

6. Verfahren nach Anspruch 1, **dadurch gekennzeichnet, daß** die Detektion der Meßsignale unter Verwendung von CCD-Kameras, Phototransistoren oder Radioaktivitäts-, Fluoreszenz- oder Luminiszenzdetektoren erfolgt.

7. Verfahren nach Anspruch 1, **dadurch gekennzeichnet, daß** derselbe Biochip, welcher mit Probenmaterial eines bestimmten Patienten beschichtet ist, zweimal oder mehrmals nacheinander einer Analyse nach den Schritten c) bis f) oder c) bis g) unterzogen wird, wobei der Biochip in der Zeit zwischen den aufeinanderfolgenden Analysen aufbewahrt wird, und wobei bei den aufeinanderfolgenden Analysen unterschiedliche Mikroareale des Biochips mit Nachweisreagenzien behandelt werden.

8. Verfahren nach einem der vorangehenden Ansprüche, **dadurch gekennzeichnet, daß** das biologische Probenmaterial direkt an die Oberfläche der Matrix des Probenträgers gebunden wird, wobei vorzugsweise die Matrix oder Oberfläche des Probenträgers durch chemische oder physikalische Verfahren vorbehandelt ist, um die Bindungsfähigkeit der Oberfläche für das Probenmaterial zu verbessern.

9. Verfahren nach einem der vorangehenden Ansprüche, **dadurch gekennzeichnet, daß** es sich bei dem an die Oberfläche gebundenen biologischen Material um Körperflüssigkeiten, vorzugsweise um Vollblut, Plasma, Serum, Urin, Ascites, Fruchtwasser, Speichel, Liquor, Lavagematerial aus Körperhohlräumen oder bronchoalveoläre Lavage, oder um Gewebe- oder Organproben, oder um Bestandteile, Zellen, Fraktionen, Konzentrate oder Extrakte aus den genannten Flüssigkeiten oder Gewebe- oder Organproben handelt.

10. Verfahren nach einem der vorangehenden Ansprüche, **dadurch gekennzeichnet, daß** die Oberfläche des Probenträgers in mindestens 100 Mikroareale pro cm² untergliedert ist, wobei die Mikroareale vorzugsweise als Vertiefungen ausgestaltet sind oder durch hydrophobe oder nicht benetzbare Grenzbereiche voneinander abgegrenzt sind.

11. Verfahren nach einem der vorangehenden Ansprüche, **dadurch gekennzeichnet, daß** die Fläche des Biochips höchstens 10 cm², vorzugsweise höchstens 4 cm² beträgt, wobei der Probenträger vorzugsweise als flächiges Gebilde, besonders bevorzugt mit quadratischem oder rechteckigem Umriß, ausgestaltet ist, und wobei die Dicke des flächigen Probenträgers vorzugsweise weniger als 3 mm, besonders bevorzugt weniger als 1 mm, beträgt.

12. Verfahren nach einem der vorangehenden Ansprüche, **dadurch gekennzeichnet, daß** die Oberfläche des Probenträgers zur Verbesserung der Bindung des Probenmaterials an besagte Oberfläche mit einer Linker-Schicht beschichtet ist, durch welche das Probenmaterial gebunden wird.

13. Verfahren nach Anspruch 12, **dadurch gekennzeichnet, daß** die Linker-Schicht aus Linker-Molekülen aufgebaut ist, welche eine selektive Bindung oder Anreicherung bestimmter Gruppen von biologischen Makromolekülen, vorzugsweise von Proteinen, Peptiden, Glycoproteinen, Zuckern, Lipiden oder Nukleinsäuren, oder die Bindung oder Anreicherung von Zellen oder bestimmten Zelltypen oder Zellpopulationen ermöglichen, wobei vorzugsweise verschiedene Teilflächen der Linker-Schicht unterschiedliche Typen von Linker-Molekülen enthalten.

14. Verfahren nach Anspruch 7 oder 8, **dadurch gekennzeichnet, daß** die Linker-Schicht auf der Oberfläche des Probenträgers diskontinuierlich ausgebildet ist, wobei Mikroareale aus linkerhaltigen Flächen und dazwischenliegenden linkerfreien Zonen gebildet werden, wobei vorzugsweise mindestens 100 Mikroareale pro cm² vorhanden sind.

15. Verfahren nach einem der vorangehenden Ansprüche, **dadurch gekennzeichnet, daß** der Probenträger mit Mitteln ausgestattet ist, die eine Identifizierung des Biochips oder die Speicherung von patientenbezogenen Daten oder die Speicherung von Analysendaten ermöglichen, vorzugsweise mittels eines maschinenlesbaren Bar-Codes, eines maschinenlesbaren Magnetstreifens, eines digitalen Speicherelements oder eines anderen maschinenlesbaren Speichermediums.

16. Verwendung eines Biochips, welcher einen Probenträger aus einer festen Matrix umfaßt, wobei die Oberfläche des Probenträgers in Mikroareale untergliedert ist und auf der Oberfläche des Biochips ein zu analysierendes Probenmaterial gebunden ist, das nur von einem einzigen, bestimmten individuellen biologischen Organismus, vorzugsweise von einem individuellen Patienten, stammt, für diagnostische Zwecke sowie zur Aufbewahrung und Archivierung des genannten Probenmaterials.

17. Verwendung eines Probenträgers aus einer festen Matrix, wobei der Probenträger eine zur Bindung von biologischem Probenmaterial geeignete, in Mikroareale untergliederte Oberfläche aufweist, zur Aufbewahrung und Archivierung von aus einem Patienten gewonnenem Probenmaterial.

18. Verwendung nach Anspruch 16 oder 17 zur Aufbewahrung und Archivierung von Patienten-Probenmaterial für Zwecke der klinischen Forschung oder bei epidemiologischen Studien, zur Qualitätssicherung und -kontrolle bei Blutbanken, Firmen oder anderen Organisationen, oder für forensische Zwecke.

## Claims

1. Diagnostic detection method comprising the following steps:
a) providing biological sample material in the form of body fluids or tissue samples from an organism to be examined, said organism being a single, specific individual biological organism, preferably an individual patient;
b) making a biochip by binding the sample material to the surface of a sample carrier by coating with the liquid or suspended sample material obtained from body fluids or cell or tissue samples, and subsequent drying, said sample carrier being a sample carrier made of a solid matrix and the surface of said sample carrier being subdivided into micro-areas;
c) applying specific, diagnostic detection reagents to single micro-areas of the biochip, using a pipetting robot, and incubation under the conditions suitable for the relevant detection reaction;
d) applying wash liquids to suppress unspecific reactions, and subsequent drawing-off of these wash liquids;
e) if necessary, repeating steps c) and d);
f) detection of measurement signals from positively reacting micro-areas;
g) computer-aided evaluation of the measurement data as well as storage of the measurement data.

2. Method according to Claim 1, **characterized in that** in step a) the biological sample material is prepared prior to its binding to the surface of the sample carrier, preferably by centrifugation, cell disintegration or extraction.

3. Method according to Claim 1, **characterized in that** steps c) to g) are performed on more than one biochip parallelly or simultaneously.

4. Method according to Claim 1, **characterized in that** the individual micro-areas of a biochip are, simultaneously or sequentially, treated with different specific diagnostic detection reagents.

5. Method according to Claim 1, **characterized in that** the specific diagnostic detection reagents utilized in step c) preferably are antibodies, antigens, lectins, DNA-probes, biomolecule-binding dyes or other specifically binding molecules.

6. Method according to Claim 1, **characterized in that** the detection of the measurement signals takes place utilizing CCD-cameras, phototransistors, or radioactivity, fluorescence or luminescence detectors.

7. Method according to Claim 1, **characterized in that** the same biochip which is coated with the sample material of a specific patient is subjected to an analysis according to steps c) to f) or c) to g), either twice or several times in succession, with the biochip being stored during the period between the successive analyses, and with different micro-areas of the biochip being treated with detection reagents in the successive analyses.

8. Method according to any one of the preceding claims, **characterized in that** the biological sample material is bound directly to the surface of the matrix of the sample carrier, with the matrix or surface of the sample carrier preferably being pre-treated by chemical or physical methods in order to improve the binding capacity of the surface for the sample material.

9. Method according to any one of the preceding claims, **characterized in that** the biological material bound to the surface are body fluids, preferably whole blood, plasma, serum, urine, ascites, amniotic water, saliva, liquor, lavage material from body cavities or bronchoalveolar lavage, or tissue samples or organ samples, or components, cells, fractions, concentrates or extracts from the liquids or tissue or organ samples mentioned.

10. Method according to any one of the preceding claims, **characterized in that** the surface of the sample carrier is subdivided into at least 100 micro-areas per cm², said micro-areas preferably being configured as depressions, or being separated from one another by hydrophobic or non-wettable border regions.

11. Method according to any one of the preceding claims, **characterized in that** the surface area of the biochip is maximally 10 cm², preferably maximally 4 cm², the sample carrier preferably being configured as a flat-shaped body, especially preferably with a square or rectangular outline, and the thickness of the flat-shaped sample carrier preferably being less than 3 mm, especially preferably less than 1 mm.

12. Method according to any one of the preceding claims, **characterized in that** to improve the binding of the sample material to the surface of the sample carrier, said surface is coated with a linker layer by means of which the sample material is bound.

13. Method according to claim 12, **characterised in that** the linker layer is composed of linker molecules which enable the selective binding or concentration of specific groups of biological macromolecules, preferably proteins, peptides, glycoproteins, sugars, lipids or nucleic acids, or the binding or concentration of cells or specific cell types or cell populations, with different surface portions of the linker layer preferably containing different types of linker molecules.

14. Method according to Claim 7 or 8, **characterized in that** the linker layer on the surface of the sample carrier is configured so as to be discontinuous, with micro-areas of linker-containing surfaces and intermediate linker-free zones being formed, and with preferably at least 100 micro-areas per cm² being present.

15. Method according to any one of the preceding claims, **characterized in that** the sample carrier is equipped with means enabling the identification of the biochip or the storage of patient-related data, or the storage of analysis data, preferably by means of machine-readable bar codes, a machine-readable magnetic strip, a digital storage element or another machine-readable storage medium.

16. Use of a biochip which comprises a sample carrier which is made of a solid matrix, the surface of the sample carrier being subdivided into micro-areas and the surface of the biochip having bound thereto a sample material to be analysed, which sample material originates exclusively from a single, specific individual biological organism, preferably an individual patient, for diagnostic purposes as well as for storing and archiving of said sample material.

17. Use of a sample carrier made of a solid matrix, said sample carrier having a surface which is suitable for binding biological sample material and which is subdivided into micro-areas, for storing and archiving of sample material collected from a patient.

18. Use according to Claim 16 or 17, for storing and archiving of patient sample material for purposes of clinical research, or in epidemiological studies, for quality assurance and quality checks in blood banks, companies or other organisations, or for forensic purposes.

## Revendications

1. Procédé de décèlement diagnostique qui comprend les étapes suivantes :
a) préparation d'une matière échantillon biologique sous la forme de liquides corporels ou d'échantillons de tissus à partir d'un organisme à examiner, à propos duquel il s'agit en l'occurrence d'un organisme biologique individuel unique déterminé, de préférence d'un patient individuel;
b) préparation d'une biopuce par liaison de la matière échantillon à la surface d'un support d'échantillon par enduction avec la matière échantillon liquide ou mise en suspension obtenue à partir de liquides corporels ou d'échantillons de cellules ou de tissus, et séchage subséquent, le support d'échantillon étant un support d'échantillon constitué d'une matrice solide et la surface du support d'échantillon étant subdivisée en microzones ;
c) application de réactifs de décèlement diagnostiques spécifiques sur des microzones individuelles de la biopuce en utilisant un robot de pipetage, et incubation dans les conditions appropriées pour la réaction de décèlement respective ;
d) application de solutions de lavage pour inhiber des réactions non spécifiques, et aspiration subséquente de ces solutions de lavage;
e) en cas de nécessité, répétition des étapes c) et d) ;
f) détection des signaux de mesure de microzones réagissant positivement ;
g) évaluation assistée par ordinateur des données de mesure, et mémorisation des données de mesure.

2. Procédé selon la revendication 1, **caractérisé en ce que**, à l'étape a), la matière échantillon biologique est préparée avant la liaison à la surface du support d'échantillon, de préférence par centrifugation, par désagrégation cellulaire ou par extraction.

3. Procédé selon la revendication 1, **caractérisé en ce que** les étapes c) à g) sont mises en oeuvre en parallèle ou de manière simultanée sur plusieurs biopuces.

4. Procédé selon la revendication 1, **caractérisé en ce que** des microzones individuelles d'une biopuce sont traitées de manière simultanée ou de manière séquentielle avec différents réactifs de décèlement diagnostiques spécifiques.

5. Procédé selon la revendication 1, **caractérisé en ce que**, à titre de réactifs de décèlement diagnostiques spécifiques à l'étape c), on met en oeuvre de préférence des anticorps, des antigènes, des lectines, des sondes d'ADN, des colorants se liant à des biomolécules ou encore d'autres molécules à liaison spécifique.

6. Procédé selon la revendication 1, **caractérisé en ce que** la détection des signaux de mesure a lieu en utilisant des caméras CCD, des phototransistors ou encore des détecteurs de radioactivité, de fluorescence ou de luminescence.

7. Procédé selon la revendication 1, **caractérisé en ce que** la même biopuce, qui a été enduite avec une matière échantillon d'un patient déterminé, est soumise à deux reprises ou plus de manière successive à une analyse après les étapes c) à f) ou c) à g), la biopuce étant conservée au cours des intervalles de temps séparant les analyses successives, et différentes microzones de la biopuce étant traitées avec des réactifs de décèlement lors des analyses successives.

8. Procédé selon l'une quelconque des revendications précédentes, **caractérisé en ce que** la matière échantillon biologique est liée directement à la surface de la matrice du support d'échantillon, la matrice ou la surface du support d'échantillon étant de préférence soumise à un prétraitement par des procédés chimiques ou physiques afin d'améliorer l'aptitude à la liaison que manifeste la surface pour la matière échantillon.

9. Procédé selon l'une quelconque des revendications précédentes, **caractérisé en ce que**, en ce qui concerne la matière biologique liée à la surface, il s'agit de liquides corporels, de préférence de sang complet, de plasma, de sérum, d'urine, d'ascite, de liquide amniotique, de salive, de liquide céphalo-rachidien, d'une matière de lavage obtenue à partir de cavités corporelles ou de lavage bronchoalvéolaire, ou encore d'échantillons de tissus ou d'organes, ou bien de constituants, de cellules, de fractions, de concentrats ou d'extraits des liquides ou bien des échantillons de tissus ou d'organes mentionnés.

10. Procédé selon l'une quelconque des revendications précédentes, **caractérisé en ce que** la surface du support d'échantillon est subdivisée en au moins 100 microzones par cm², les microzones étant de préférence réalisées sous la forme de renfoncements ou bien étant délimitées les unes par rapport aux autres par des zones de délimitation hydrophobes ou non mouillables.

11. Procédé selon l'une quelconque des revendications précédentes, **caractérisé en ce que** la surface de la biopuce s'élève au maximum à 10 cm², de préférence au maximum à 4 cm², le support d'échantillon étant de préférence réalisé sous la forme d'un produit plan, de manière particulièrement préférée avec un contour carré ou rectangulaire, et l'épaisseur du support plan d'échantillon étant de préférence inférieure à 3 mm, de manière particulièrement préférée inférieure à 1 mm.

12. Procédé selon l'une quelconque des revendications précédentes, **caractérisé en ce que** la surface du support d'échantillon est enduite d'une couche de lieur pour améliorer la liaison de la matière échantillon à ladite surface, couche par laquelle on obtient la liaison de la matière échantillon.

13. Procédé selon la revendication 12, **caractérisé en ce que** la couche de lieur est constituée par des molécules faisant office de lieurs qui permettent d'obtenir une liaison sélective ou un enrichissement sélectif de groupes déterminés de macromolécules biologiques, de préférence de protéines, de peptides, de glycoprotéines, de sucres, de lipides ou d'acides nucléiques, ou encore la liaison ou l'enrichissement de cellules ou bien de types de cellules déterminés ou de populations cellulaires déterminées, différentes surfaces partielles de la couche de lieur contenant de préférence des types différents de molécules faisant office de lieurs.

14. Procédé selon la revendication 7 ou 8, **caractérisé en ce que** la couche de lieurs est réalisée en discontinu sur la surface du support d'échantillon, des microzones étant formées par des surfaces contenant un lieur et par des zones intermédiaires exemptes de lieur, au moins 100 microzones étant de préférence présentes par cm².

15. Procédé selon l'une quelconque des revendications précédentes, **caractérisé en ce que** le support d'échantillon est équipé de moyens qui permettent de procéder à une identification de la biopuce ou de mémoriser des données concernant le patient ou encore de mémoriser des données analytiques, de préférence au moyen d'un code à barres lisible par machine, d'une bande magnétique lisible par machine, d'un élément d'enregistrement numérique ou d'un autre support d'enregistrement lisible par machine.

16. Utilisation d'une biopuce qui comprend un support d'échantillon constitué d'une matrice solide, la surface du support d'échantillon étant subdivisée en microzones et une matière échantillon à analyser étant liée à la surface de la biopuce, ladite matière provenant seulement d'un organisme biologique individuel unique déterminé, de préférence d'un patient individuel, à des fins diagnostiques et pour la conservation et l'archivage de la matière échantillon mentionnée.

17. Utilisation d'un support d'échantillon constitué d'une matrice solide, le support d'échantillon présentant une surface subdivisée en microzones, appropriée pour la liaison d'une matière échantillon biologique, pour la conservation et l'archivage d'une matière échantillon provenant d'un patient.

18. Utilisation selon la revendication 16 ou 17 pour la conservation et l'archivage d'une matière échantillon d'un patient à des fins de recherche clinique ou dans le cadre d'études épidémiologiques, pour la sécurité et le contrôle de la qualité dans des banques de sang, dans des firmes ou dans d'autres organisations, ou à des fins médico-légales.
